# EUROPÄISCHE PATENTSCHRIFT

(11) **EP 1 904 432 B1**
(45) Veröffentlichungstag und Bekanntmachung des Hinweises auf die Patenterteilung: **02.03.2011**
(21) Anmeldenummer: 06776195.7
(22) Anmeldetag: 11.07.2006
(51) Int. Cl.: A61P 17/00, A61K 31/194, A61K 31/201, A61K 31/20, A61K 31/202, A61K 31/23, A61K 31/231, A61K 31/232, A61K 31/24, A61K 31/164

(54) **OLIGOMERE GERADKETTIGER UND UNVERZWEIGTER FETTSAÜREN UND DIESE ENTHALTENDE ARZNEIMITTEL**
OLIGOMERS OF STRAIGHT-CHAIN AND UNBRANCHED FATTY ACIDS, AND MEDICAMENTS CONTAINING THE SAME
OLIGOMERES D'ACIDES GRAS A CHAINES LINEAIRES ET NON RAMIFIES, ET MEDICAMENTS RENFERMANT DES COMPOSES

(30) Priorität: 11.07.2005 DE 102005032307
(43) Veröffentlichungstag der Anmeldung: 02.04.2008
(60) Teilanmeldung: 10005521.9
(73) Patentinhaber: PLT Patent & Licence Trading Ltd., c/o The B-Net Sheraton House Castle Park Cambridge CB3 0AX (GB)
(72) Erfinder: WOLF, Hans-Uwe, 89231 Neu-Ulm (DE)
(74) Vertreter: Pfenning, Meinig & Partner GbR
(86) Internationale Anmeldenummer: PCT/EP2006/006773
(87) Internationale Veröffentlichungsnummer: WO 2007/006550

(56) Entgegenhaltungen:
- WO-A-93/14060
- DE-A1- 10 305 965
- DATABASE CA [Online] CHEMICAL ABSTRACTS SERVICE, COLUMBUS, OHIO, US; ONO, SEIGO ET AL: "Nitroaniline-type nonlinear optical material" XP002404917 gefunden im STN Database accession no. 110:104607 & JP 63 210918 A2 (OKI ELECTRIC INDUSTRY CO., LTD., JAPAN) 1. September 1988 (1988-09-01)
- WARWEL S ET AL: "SURFACTANTS FROM GLUCAMINES AND OMEGA-EPOXY FATTY ACID ESTERS" EUROPEAN JOURNAL OF LIPID SCIENCE AND TECHNOLOGY, WILEY VCH VERLAG, WEINHEIM, DE, Bd. 103, Nr. 10, Oktober 2001 (2001-10), Seiten 645-654, XP001101948 ISSN: 1438-7697
- DATABASE WPI Week 2005 Derwent Publications Ltd., London, GB; AN 2005-310723 XP002405079 & JP 2005 104928 A (BUNSHI SEIRI KAGAKU KENKYUSHO KK) 21. April 2005 (2005-04-21)
- DATABASE WPI Week 2004 Derwent Publications Ltd., London, GB; AN 2004-521523 XP002405080 & JP 2004 196665 A (POLA CHEM IND INC) 15. Juli 2004 (2004-07-15)

## Beschreibung

Die vorliegende Erfindung betrifft neue Substanzen, die sich aus natürlicherweise vorkommenden geradkettigen und unverzweigten Fettsäuren sowie aus halbsynthetischen und synthetischen Verbindungen mit prinzipiell gleicher Struktur dadurch ableiten, dass sie Dimere der Ausgangssubstanzen darstellen.

Die Erfindung ist durch den Geltungsbereich der Anspruche definiert.

Grundsätzlich enthalten alle biologischen Membranen, insbesondere Zellmembranen, als essentielle Bestandteile sog. Lipide und Lipid-analoge Substanzen, die strukturell unterschiedlich aufgebaut sind, die sich jedoch in ihrer prinzipiellen Bauweise ähneln. Die prinzipielle Ähnlichkeit der Struktur besteht darin, dass sie aus einem hydrophoben und einem hydrophilen Anteil aufgebaut sind.

Im Fall der Lipid-analogen Substanzen aus der Gruppe der geradkettigen und unverzweigten Fettsäuren wie z.B. der Palmitinsäure besteht der hydrophobe Molekülbereich aus einem Fettsäurerest, während der hydrophile Anteil eine Carboxylgruppe ist.

Die amphiphile Struktur der Lipid-analogen Substanzen, d.h. die gleichzeitige Anwesenheit eines (stark) hydrophoben und eines hydrophilen, polaren Anteils der Molekülstruktur, führt dazu, dass sich die Lipid-analogen Substanzen in einer wässrigen Phase (meist zusammen mit anderen Lipiden) spontan zu einer Lipid-Doppelschicht, einem so genannten "Lipid-Bilayer" anordnen, der u. a. die Grundlage der Struktur biologischer Membranen darstellt. Das Bauprinzip dieser Doppelschicht ist für alle Lipide und Lipid-analogen Substanzen gleich: Sie ordnen sich in zwei parallelen, eng zusammenliegenden Schichten an, wobei sich jeweils die hydrophoben Reste der betreffenden Moleküle direkt gegenüberliegen und in Kontakt treten. Sie bilden somit den hydrophoben inneren Bereich der Membrandoppelschicht, während die hydrophilen Reste auf beiden Seiten der Lipid-Doppelschicht mit der wässrigen Phase in Kontakt stehen. Die Tendenz zur Ausbildung dieser Lipid-Doppelschicht besteht sowohl innerhalb als auch außerhalb eines Organismus, z. B. in einem wässrigen System, in welchem die Eigenschaften der Lipid-Doppelschichten in eigens hierzu ausgelegten experimentellen Anordnungen untersucht werden können.

Obwohl sich die Struktur der Lipid-Doppelschicht in einem Organismus spontan ausbildet und eine erhebliche Stabilität besitzt, besteht z. B. bei Vorliegen einer Lipid-Stoffwechselstörung die Möglichkeit, dass eine biologische Membran einen Teil ihrer Lipid-Komponenten verliert, weil diese Moleküle entweder zu langsam und/oder in einem unzureichenden Ausmaß gebildet oder (zu schnell) verstoffwechselt werden und damit der Membranstruktur entzogen werden, wobei die betreffenden Membranen an den jeweiligen Komponenten verarmen. Dies führt u. a. zu einer Störung der Membranstruktur und -funktion. Ein bekanntes Beispiel für derartige Veränderungen ist die Verarmung der Lipid-Doppelschichten des Stratum corneum der menschlichen Haut an Fettsäuren. In verschiedenen Arbeiten wurde nachgewiesen, dass z.B. Palmitinsäure fester Bestandteil des Stratum corneum ist und für die normale Barriere-Funktion der Haut erforderlich ist (Man MQM, Feingold KR, Thornfeldt CR, Elias PM (1996): J Invest Dermatol 106 (5): 1096-1101; Mao-Qiang M, Elias PM, Feingold KR (1993): J Clin Invest 92: 791-798; Mao-Qiang M, Jain M, Feingold KR, Elias PM (1996): J Invest Dermatol 106 (1): 57-63; Velkova V, Lafleur M (2002) : Chem Phys Lipids 117 (1-2): 63-74.

Verschiedene Hautveränderungen und Hauterkrankungen basieren auf den Veränderungen der Lipid-Zusammensetzung der stratum-corneum-schicht in der menschlichen Haut. Diese Veränderungen der Lipid-Zusammensetzung führen zu einer mehr oder weniger stark veränderten Barrierefunktion der betroffenen Hautpartien für Wasser (McIntosh TJ, Stewart ME, Downing DT (1996): Biochemistry 35(12): 3.649-3.653; Coderch L, de Pera M, Perez-Cullell N, Estelrich J, de la Maza A, Parra JL (1999): Skin Pharmacol Appl Skin Physiol 12 (5): 235-246). Hautveränderungen und Hauterkrankungen dieser Art sind beispielsweise:
1. Atopische Dermatitis
2. "trockene" Haut-Xerose, Xerodermie
3. dyshidrotisches Ekzem
4. chronisches kumulativ-toxisches Kontaktekzem
5. alternde Haut
6. durch UV-Licht stark beanspruchte Haut
7. Sebostase
8. Verhornungsstörungen

Insbesondere auf dem Gebiet der Klinischen Medizin ist es in den genannten Fällen von Erkrankungen wünschenswert, die Struktur der im Organismus vorhandenen biologischen Membran, i. e. der Lipid-Doppelschichten, in geeigneter Weise zu verändern und/oder zu stabilisieren.

Wie oben bereits ausgeführt, sind biologische Membranen sehr vieler Zellen aus einer Lipid-Doppelschicht aufgebaut, die eine wirksame Barriere gegenüber dem Extrazellularraum darstellt. Dies trifft auch für das Stratum corneum der Haut zu. Beim Menschen besteht diese Hautstruktur aus mehreren Schichten keratinisierter Corneocyten, die in eine Lipidmatrix einer stark geordneten lamellaren Struktur eingebettet sind. Diese Lipid-Doppelschichten enthalten im Wesentlichen Ceramide, Fettsäuren, wie z.B. Palmitinsäure, sowie Cholesterol (Cholesterin).

Gemäß neueren Erkenntnissen zum Pathomechanismus der Atopischen Dermatitis (Arikawa J, Ishibashi M, Kawashima M, Takagi Y, Ichikawa Y, Imokawa G (2002) : J Invest Dermatol 119(2): 433-439) und verwandter Erkrankungen ist die Ursache für die Anfälligkeit der Haut im Fall einer derartigen Erkrankung u. a. ein veränderter Lipidstoffwechsel bzw. reduzierter Lipid-Gehalt des Stratum corneum. Diese Veränderungen betreffen u.a. den Fettsäure-Stoffwechsel. So zeigten sich bei der Atopischen Dermatitis, aber auch bei anderen Hauterkrankungen wie bei Psoriasis, bei (lamellarer) Ichthyosis und bei Kontaktdermatitis in der Haut der Patienten erniedrigte Gehalte an freien Fettsäuren (Pilgram GS, Vissers DC, van der Meulen H, Pavel S, Lavrijsen SP, Bouwstra JA, Koerten HK (2001) : J Invest Dermatol 117 (3): 710-717) .

Die physiologische Zusammensetzung der Membran-Lipide des Stratum corneum der menschlichen Haut ist allerdings noch aus einem zweiten Grund für die normale Struktur und Funktion der Haut von essentieller Bedeutung. Die Anwesenheit eines ausreichenden Gehalts an diesen Lipiden gewährleistet die uneingeschränkte Fähigkeit der Haut zur Bindung einer physiologischen Menge an Wasser. Der Verlust eines Teils der Stratum-corneum-Lipide führt daher zu einer Einschränkung der Wasserbindefähigkeit, dem so genannten transepidermalen Wasserverlust der Haut. Dies zeigt sich im Auftreten einer "trockenen" und faltigen Haut, die häufig insbesondere, aber nicht ausschließlich, im höheren Lebensalter auftritt.

Die heutigen Möglichkeiten, die Symptome und Folgen der genannten Hautkrankheiten, insb. der Atopischen Dermatitis, zu lindern (von einer Heilung kann derzeit noch keine Rede sein), sind nach wie vor noch sehr begrenzt. Die topische Anwendung spezieller Glucocorticoide und immunsuppressiver Wirkstoffe ist wegen der Toxizität dieser Substanzen mit erheblichen Risiken verbunden. Bestimmte Corticoide verursachen sogar einen geradezu kontraproduktiven Effekt, indem sie zu einem Verlust an Ceramiden, Cholesterol und freien Fettsäuren führen.

Unter Berücksichtigung des heutigen Kenntnisstands über die Bedeutung einer physiologischen Lipid-Zusammensetzung der Stratum-corneum-Membranen ist es folgerichtig, dass versucht wird, die im Stratum corneum vorhandenen Defizite an Membran-Lipiden durch exogene Zufuhr auszugleichen, In der Praxis versucht man daher, mit Hilfe von Salben, Cremes und dergleichen die fehlenden Lipide, z.B. freie Fettsäuren, der veränderten bzw. erkrankten Haut zuzuführen. Dies erfolgt beispielsweise durch speziell hierfür formulierte Lipid-Präparate unter Einschluss von freien Fettsäuren, u. a. durch die Verwendung von Liposomen. Zahlreiche Produkte sind zur Therapie der genannten Hautkrankheiten inzwischen auf dem Markt.

Die hier geschilderten therapeutischen Maßnahmen sind sicherlich als prinzipiell richtig anzusehen, da sie in logischer Weise die vorhandenen Defizite des Stratum corneum an Lipiden und Lipid-analogen Substanzen auszugleichen versuchen. Die während der letzten Jahre mit diesen therapeutischen Maßnahmen gemachten Erfahrungen zeigen jedoch, dass trotz der prinzipiellen Richtigkeit des therapeutischen Ansatzes die Ergebnisse dieser Heilbehandlungen keineswegs überzeugend sind. Zum Teil ist der Erfolg der durchgeführten Maßnahmen unsicher. Selbst dann, wenn sich ein annähernd akzeptabler Erfolg der Heilbehandlung einstellt, hat eine derartige Heilbehandlung zumindest zwei gravierende Nachteile:
- Das Ausmaß des Heilerfolgs ist nicht so groß, dass von einer vollständigen Gesundung der erkrankten Haut gesprochen werden kann.
- Um einen einigermaßen akzeptablen Heilerfolg an der Haut über einen längeren Zeitraum zu gewährleisten, müssen die genannten Lipide und Lipid-analogen Substanzen in kurzen Zeitabständen permanent der Haut zugeführt werden.

Beide Nachteile sind auf eine gemeinsame Ursache zurückzuführen. Die genannten freien Fettsäuren sind keine statischen Komponenten der Haut, sondern sie sind Zwischenprodukte einer Reaktionsfolge, in der die von der Haut benötigten freien Fettsäuren z.B. aus Nahrungsfetten (Triglyceriden), aus Ceramiden oder Phospholipiden freigesetzt werden und nach ihrer Freisetzung zur Wahrnehmung ihrer Funktion als Membranbestandteil des Stratum corneum anschließend in den oxidativen Fettsäure-Stoffwechsel eingeschleust werden.

Diese Reaktionsabfolge stellt ein Fließgleichgewicht dar, in welchem eine gewisse Menge der genannten Komponenten durch die Wirkung bestimmter Enzyme von Stufe zu Stufe metabolisch verändert wird. Es liegt somit ein gewisser Durchsatz an Substanz vor. Die als Hauttherapeutica exogen zugeführten Fettsäuren werden in diese Reaktionsabfolge eingeschleust. Liegt *a priori* eine Störung dieser Reaktionsabfolge vor, die dann zu einer pathologischen Lipid-Zusammensetzung des Stratum corneum führt, so ist zu erwarten, dass die exogene Zufuhr von Lipiden in Form eines Therapeuticums an diesem pathologischen Zustand grundlegend nichts oder nicht viel ändern kann, da der exogen zugeführte Fettsäure-Anteil vom Organismus in gleicher Weise weiterverarbeitet wird, wie dies bei dem endogen zur Verfügung gestellten Fettsäure-Anteil der Fall ist. Ein Heilerfolg ist daher mit den derzeit zur Verfügung stehenden therapeutischen Möglichkeiten in hohem Maße davon abhängig, dass die betreffenden therapeutischen Ersatzstoffe schneller in die Haut eindringen können als sie in die vorhandenen physiologischen Abbauschritte eingeschleust werden, und dass sie über einen längeren Zeitraum, im Extremfall lebenslang, kontinuierlich zugeführt werden.

Das vorliegende Problem ist nicht ohne weiteres lösbar. Der Geschwindigkeit der Aufnahme von Lipiden und Lipid-analogen Substanzen in das Stratum corneum hinein sind gewisse physiologische und physikalischchemische bzw, biochemische Grenzen gesetzt, z. B. hinsichtlich der Diffusionsgeschwindigkeit der Therapeutica. Diese Geschwindigkeit kann nicht beliebig gesteigert werden. Zum anderen sind die an den genannten Reaktionsfolgen beteiligten lipidabbauenden Enzyme durch exogene Maßnahmen nicht oder nicht ohne gravierende Probleme im Sinne einer Minderung ihrer Aktivität zu beeinflussen.

Der komplementäre therapeutische Ansatz, i.e., die Aktivierung lipidsynthetisierender Enzyme durch exogene Wirkstoffe (z.B. Nicotinamid), ist nur in begrenztem Umfang möglich und bisher lediglich *in vitro* gelungen (Tanno O, Ota Y, Kitamura N, Katsube T, Inoue S (2000) : British J Dermatol 143 (3) : 524-531) .

Warwel et al., Eur. J. Lipid Sci. Technol. 103 (2001), S. 645-654, beschreibt Dimere der Rezinolsäure, die über N-Methylglucamin als Spacer verbunden sind.

Die JP 2005/104928 A beschreibt Hautpflegemittel gegen trockene Haut und Falten. Die hier eingesetzten Verbindungen basieren auf Tryglyceriden mit Fettsäuren mit einer Kettenlänge im Bereich von 8 bis 12 C-Atomen.

Die DE 103 05 965 A1 beschreibt kosmetische oder dermatologische Zubereitungen, die 8-Hexadecen-1,16-dicarbonsäure enthalten.

Zur Lösung des beschriebenen Problems ist es erforderlich, grundsätzlich andere Prinzipien anzuwenden, um die therapeutische Wirksamkeit exogen zugeführter Lipid-Ersatzsubstanzen oder -Analogsubstanzen zu erhöhen.

Die Aufgabe der vorliegenden Erfindung ist es deshalb, Verbindungen bereitzustellen, durch die im Organismus vorhandene biologische Membranen verändert und/oder stabilisiert werden können.

Diese Aufgabe wird in Bezug auf das Kosmetik- oder Körperpflegemittel sowie in Bezug auf die Verwendung als Arzneimittel durch die Merkmale des Anspruchs 9 gelöst. Die weiteren abhängigen Ansprüche zeigen vorteilhafte Weiterbildungen auf.

Unter Oligomeren wird die Verknüpfung von zwei bis zwölf Monomeren verstanden. Bevorzugt sind hier insbesondere Dimere, Trimere, Tetramere, Pentamere, Hexamere und Octamere.

Der Begriff "Dimerisierung" wird gemäß der vorliegenden Erfindung auch dann verwendet, wenn es sich nicht nur um die direkte kovalente Verknüpfung zweier Moleküle unter Verdopplung der Zahl der jeweils enthaltenen Atome handelt, sondern auch dann, wenn die beiden ursprünglichen Einzelmoleküle durch eine kurze Molekülbrücke im Sinne eines sog. Spacers verbunden sind. Der Begriff "Oligomere" wird auch dann verwendet, wenn es sich bei diesen Verbindungen nicht nur um mehrere kovalente verknüpfte Monomere handelt, sondern auch, wenn die Monomere durch Molekülbrücken in Form unterschiedlicher Spacer verbunden sind.

Die Bausteine der Lipid-analogen Substanzen aus der Gruppe der Fettsäuren bestehen dabei bevorzugt aus Palmitinsäure oder aus anderen Monocarbonsäuren mit einer Kettenlänge zwischen 10 und 40 C-Atomen. Bevorzugt sind die Fettsäuren ausgewählt aus der Gruppe bestehend aus n-Hexadecansäure (Palmitinsäure, C₁₅H₃₁-COOH), n-Dodecansäure (Laurinsäure, C₁₁H₂₃-COOH), n-Tetradecansäure (Myristicinsäure, C₁₃H₂₇-COOH), n-Octadecansäure (Stearinsäure, C₁₇H₃₅-COOH), n-Eicosansäure (Arachinsäure, C₁₉H₃₉-COOH), n-Tetracosansäure (Lignocerinsäure, C₂₃H₄₇-COOH), 9-Hexadecensäure (Palmitoleinsäure, C₁₅H₂₉-COCH), 9-Octadecensäure (Oleinsäure, Ölsäure, C₁₇H₃₃-COOH), 9,11-Octadecadiensäure (C₁₇H₃₁-COON), 9,12-Octadecadiensäure (Linolsäure, C₁₇H₃₁-COOH) , 9,12,15-Octadecatriensäure (Linolensäure, C₁₇H₂₉-COOH), 5,8,11,14,17-Eicosapentaensäure ("EPA", C₁₉H₂₉-COOH), 4,7,10,13,16,19-Docosahexaensäure ("DHA", C₂₁H₃₁-COOH) , Decansäure (C₁₀H₂₁-COOH), Octacosansäure (C₂₈H₅₇-COOH) und 9-Octacosensäure (C₂₈H₅₅-COOH).

Die erfindungsgemäßen Verbindungen müssen den folgenden Anforderungen genügen:
1. Bei den die Dimere bildenden Fettsäuren muss es sich um geradkettige und unverzweigte Verbindungen handeln.
2. Die Dimerisierung muss unter Ausbildung ausschließlich kovalenter Bindungen zwischen den einzelnen Fettsäuren zustande kommen.
3. Die grundsätzliche Struktur der eingesetzten Lipide oder Lipid-analogen Substanzen, welche die Ausbildung der Lipid-Doppelmembran ermöglicht, soll nicht nur erhalten bleiben, sondern die Fähigkeit zur Ausbildung der Doppelmembran soll verstärkt werden, weil die durch die genannten Erkrankungen geschädigte Haut ohnehin nur eine eingeschränkte Fähigkeit zum Aufbau und zum Erhalt der physiologischen Lipid-Doppelmembran besitzt.
4. Die Struktur der eingesetzten Lipide oder Lipid-analogen Substanzen soll bei erhaltener Grundstruktur so verändert werden, dass sie nur noch in geringerem Umfang als Substrate der in der Haut, speziell im Stratum corneum, vorhandenen Enzyme fungieren können. Dies bedeutet, dass sie in deutlich geringerem Umfang als die originären Lipide oder Lipid-analogen Substanzen in die jeweiligen enzymatischen Reaktionsfolgen eingeschleust werden sollen und somit als wesentliche strukturelle Bestandteile des Stratum corneum über einen längeren Zeitraum als die originären Lipide bzw. Lipid-analogen Substanzen erhalten bleiben sollen.
5. Die Abänderung der Molekülstruktur soll jedoch nur in einem so geringen Umfang erfolgen, dass durch den geringen stoffwechselbedingten Um- oder Abbau der zugeführten dimeren Zusatz-Lipide solche Substanzen entstehen, die den körpereigenen Lipiden oder Lipid-analogen Substanzen möglichst ähnlich sind. Auf diese Weise wird die Gefahr erheblich verringert, dass Stoffwechselprodukte mit toxischer Wirkung entstehen.

Der Aufbau einer Molekülstruktur, welche die drei oben angegebenen Forderungen erfüllt, besteht zunächst in einer Dimerisierung der für die erwartete therapeutische Wirksamkeit eingesetzten Fettsäuren.

Wegen der strukturellen Asymmetrie der gesamten Gruppe der Fettsäuren - auf der einen Seite der/die Fettsäurerest(e) als hydrophober Strukturanteil (im englischen Sprachgebrauch als "tail" bezeichnet) und auf der anderen Seite der hydrophile Rest in Form der Carboxylgruppe (im englischen Sprachgebrauch als "head" bezeichnet) - lassen sich drei prinzipiell verschiedene Arten unterscheiden, wie zwei monomere Fettsäure-Moleküle zu einem dimeren Molekül kovalent verbunden werden können:
1. in Form einer "tail-to-tail"-Anordnung, d. h. durch kovalente Verknüpfung zwischen den hydrophoben Fettsäureresten der beiden zu verbindenden Moleküle. Diese Verknüpfung erfolgt z. B. durch den Einbau eines sog. intramembranären Spacers.
2. in Form einer "head-to-head"-Anordnung, d. h. durch kovalente Bindung zwischen den beiden hydrophilen, polaren Carboxylgruppen der beiden zu verbindenden Fettsäure--Moleküle. Diese Verknüpfung erfolgt z.B. durch den Einbau eines sog. extramembranären Spacers.
3. in Form einer "head-to-tail"-Anordnung, d. h. durch kovalente Bindung zwischen der Carboxylgruppe des einen Fettsäure-Moleküls und dem hydrophoben Fettsäurerest des zweiten Fettsäure-Moleküls.

Die erfindungsgemäße Variante 1, d.h. die tail-to-tail-Anordnung, basiert auf der Verknüpfung vorzugsweise der jeweils ω-ständigen Kohlenstoffatome der Fettsäurereste der beiden zusammenzufügenden Moleküle mit Hilfe eines Spacers. Ein derartiger Spacer kann zum einen wegen der Verknüpfung zweier Monomerer zu einem Dimer als "intradimerer" Spacer bezeichnet werden. Da dieser Spacer bei Einlagerung des Dimers in die biologische Membran im Membraninnern angeordnet ist, wird er vorzugsweise als "intramembranärer" Spacer bezeichnet. Die Bezeichnungen intradimer und intramembranär sind somit von ihrer Bedeutung gleichzusetzen.

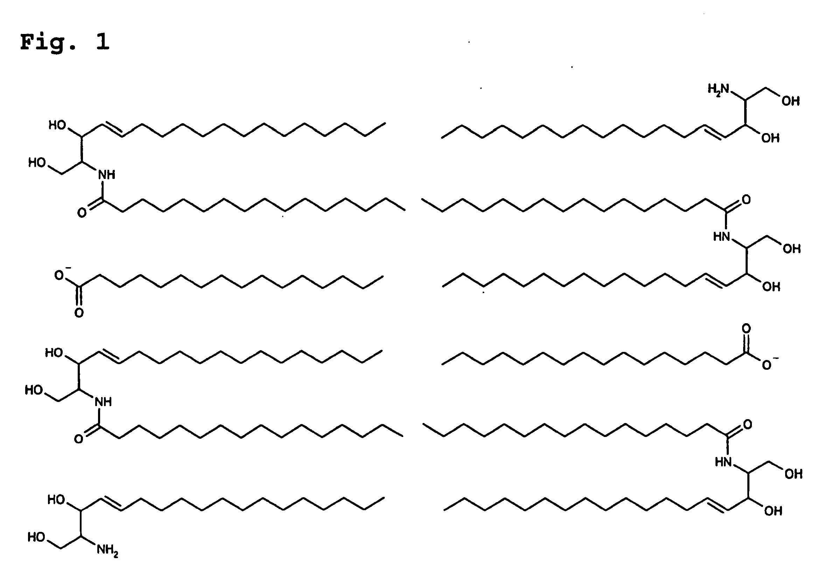

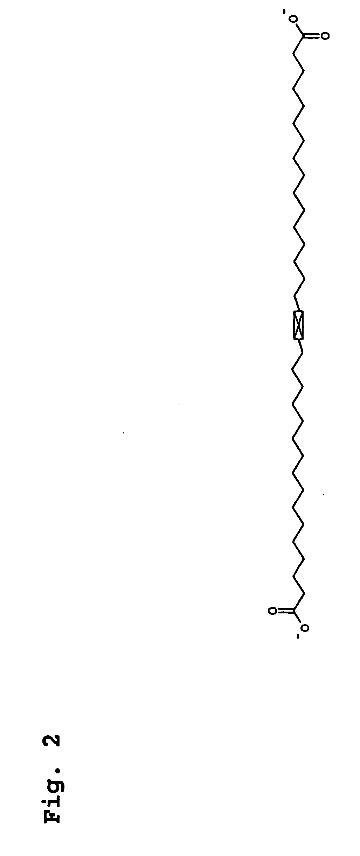

Dieser intramembranäre Spacer muss, da er sich im hydrophoben Innenbereich der biologischen Membran befindet, eine hydrophobe Natur besitzen. Somit liegt ein dimeres Molekül vor, das auf Grund der Anordnung seines hydrophoben Molekülbereichs im Innern des Dimers problemlos in eine biologische Lipid-Doppelschicht zu integrieren ist. Fig. 2 zeigt die Art der Verknüpfung und die Ähnlichkeit des Dinterisierungsprodukts mit der physiologischen Struktur der Lipid-Doppelmembran am Beispiel zweier Fettsäure-Moleküle (vgl. Fig. 1) .

Die tail-to-tail-Anordnung stellt die unmittelbare Nachahmung der natürlicherweise in den biologischen Membranen vorhandenen stabilen Anordnung der Fettsäuren dar, wie durch einen Vergleich mit der Anordnung der Komponenten in Fig. 1 zu erkennen ist. Das tail-to-tail-Dimere ist als eine von zwei möglichen Grundstrukturen für die Gesamtheit aller weiteren hier beschriebenen Lipid-Oligomere anzusehen.

Die Dimerisierung der Fettsäuren durch Verknüpfung über einen intramembranären Spacer führt somit zu Molekülen, die sich nicht nur in die Struktur einer Lipid-Doppelmembran ohne Probleme einfügen, sondern die auch darüber hinaus durch die Anwesenheit einer kovalenten Verknüpfung über die ω-ständigen C-Atome der Fettsäurereste zweier sich gegenüberliegender Fettsäure-Moleküle zu einer erheblichen Strukturstabilisierung der Lipid-Doppelmembran beitragen.

Die Variante 2, i. e. das head-to-head-Dimere, besitzt eine Struktur, die eine Integration des Moleküls in nur eine einzige Lipid-Doppelschicht nicht erlaubt, weil der hydrophile Bereich dieses dimeren Moleküls im hydrophoben Inneren der Membrandoppelschicht angeordnet wäre, was eine äußerst instabile Struktur darstellen würde, die sich demzufolge nicht spontan ausbildet. Die head-to-head-Dimeren haben jedoch insofern eine biologische oder medizinische Bedeutung, als die beiden derart verknüpften Fettsäure-Moleküle in zwei parallel in nahem Abstand angeordneten Lipid-Doppelschichten verankert sein können, wobei sich jedes der beiden Monomere in jeweils einer Hälfte der beiden parallelen Lipid-Doppelschichten befindet. Solche in nahem Abstand angeordnete Lipid-Doppelschichten kommen z.B. in der Markscheide von Nervenzellen vor.

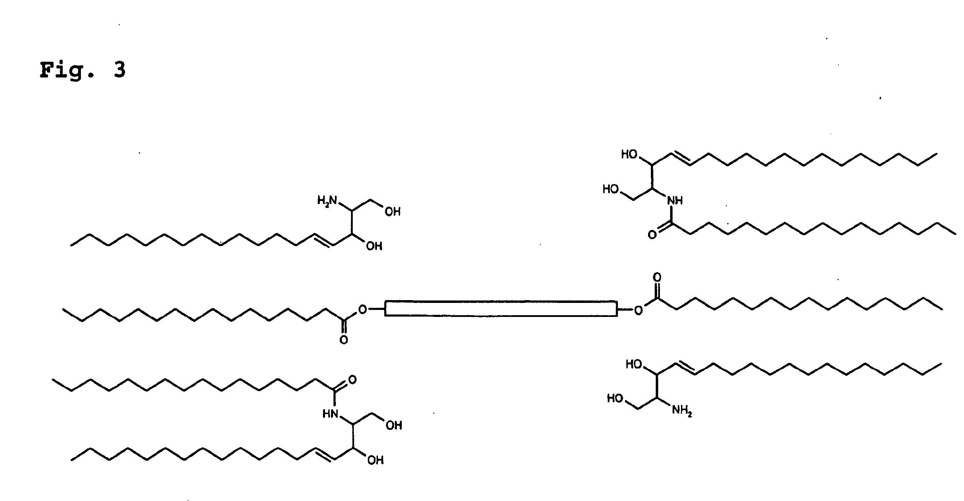

Im Fall des "head-to-head"-Fettsäure-Dimers kann es allerdings aus räumlichen Gründen erforderlich sein, zwischen die beiden Monomere einen sog. Spacer (mit variabler Kettenlänge) einzubauen, um eine Integration der beiden Lipid-Komponenten in die beiden Lipid-Doppelschichten auch dann zu ermöglichen, wenn diese Doppelschichten einen gewissen Abstand voneinander haben (s. hierzu Fig. 3). Dies trifft z.B. bei den parallel angeordneten Lipid-Doppelschichten des Stratum corneum der menschlichen Haut zu.

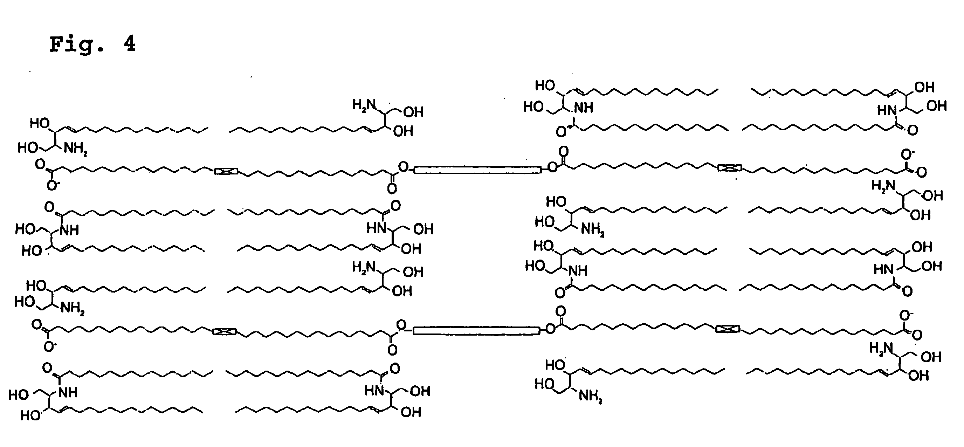

Allerdings ist davon auszugehen, dass sich ein solches Molekül noch nicht in optimaler Weise in zwei parallel angeordnete Lipiddoppelschichten integriert. Die optimale Integration wird durch ein Molekül erreicht, bei dem zwei Dimere (anstelle der beiden Monomere) miteinander verknüpft werden, die dann in den beiden parallel angeordneten biologischen Membranen integriert sind. Dieses (tetramere) Molekül hat dann die folgenden Strukturmerkmale (s. hierzu Fig. 4):

Die beiden Dimere sind jeweils aus den Monomeren in der tail-to-tail-Anordnung durch Verknüpfung mit Hilfe des oben erwähnten intramembranären Spacers aufgebaut. Die Verknüpfung der beiden Dimere erfolgt in der head-to-head-Anordnung über einen weiteren Spacer, der als interdimerer Spacer bezeichnet werden kann, weil er zwischen zwei präformierten Dimeren angeordnet ist. Da er sich nach der Integration des Gesamtmoleküls außerhalb der beiden Membranen befindet, wird er jedoch vorteilhafter als extramembranärer Spacer bezeichnet. Die beiden Bezeichnungen interdimer und extramembranär sind somit von ihrer Bedeutung gleichzusetzen.

Der extramembranäre Spacer muss wegen seiner Lage außerhalb der Membran, d.h., im hydrophilen extramembranären Bereich der Zelle, eine hydrophile Struktur besitzen.

Die Variante 3 der Lipid-Dimerisierung hat praktisch keine biologische oder medizinische Bedeutung, da sich ein Molekül dieser Struktur (ohne oder mit Spacer) nicht in irgendeiner Weise in eine oder zwei parallel angeordnete Lipid-Doppelschichten integrieren lässt. In allen Fällen müssten zumindest zum Teil hydrophile Strukturanteile des Dimers in hydrophobe Bereiche der Membranen integriert werden, was bekanntermaßen zu sehr instabilen Strukturen führen würde, die sich aus diesem Grunde nicht spontan bilden können.

Es besteht auch die Möglichkeit, Hybridmoleküle z.B. aus je einem Molekül Palmitinsäure und einem Molekül einer anderen gesättigten oder ungesättigten Fettsäure oder aus zwei Molekülen der genannten Fettsäuren gemäß dem Anspruch 3 herzustellen und in der unten beschriebenen Weise zu therapeutischen Zwecken einzusetzen.

Durch die Dimerisierung und besonders durch die Oligomerisierung ist die Gewähr gegeben, dass ein derartiges Molekül durch die in der Haut vorhandenen Enzyme des Fettsäure-Stoffwechsels sehr viel langsamer ab- bzw. umgebaut wird, als dies für die korrespondierenden monomeren Fettsäuren zutrifft. Die mit der Dimerisierung bzw. Oligomerisierung verbundene Vergrößerung des Fettsäure-Moleküls führt zu einer starken Minderung der enzymatisch gesteu-erten Metabolisierung, weil bei der bekanntermaßen hohen Substratspezifität der meisten Enzyme die Größenänderung eines Substrats um den Faktor von mindestens 2 die Geschwindigkeit des Substratumsatzes erheblich abfallen lässt.

Andererseits sind die entstehenden Reaktionsprodukte von ihrem allgemeinen Aufbau her den natürlicherweise vorkommenden Molekülen des Fettsäure-Stoffwechsels so ähnlich, dass ein Einschleusen in die entsprechenden Reaktionsfolgen ohne Probleme möglich ist. Darüber hinaus ist auch in keiner Weise damit zu rechnen, dass die dimerisierten bzw. oligomerisierten Fettsäure-Moleküle wegen der großen Ähnlichkeit mit physiologischerweise vorkommenden Molekülspezies eine relevante Toxizität besitzen.

Ein gewisser Grad der physiologischen Abbaubarkeit der Fettsäure-Dimere und -Oligomeres die allerdings als deutlich geringer anzusehen ist als die der monomeren Fettsäure-Moleküle, ist somit eine aus pharmakokinetischen und pharmakologischen Gründen erwünschte Eigenschaft des Moleküls, weil hierdurch die Steuerbarkeit der Therapie besser gewährleistet ist, als wenn keinerlei metabolischer Abbau mehr möglich wäre.

Für den Fall, dass die aus den Fettsäuren durch Verknüpfung der beiden ω-ständigen C-Atome der Fettsäurereste entstehenden Produkte eine zu geringe metabolische Abbaubarkeit besitzen, kann eine ausreichend hohe Abbaubarkeit dadurch erreicht und gewährleistet werden, dass in den oben erwähnten intramembranären Spacer quasi eine "metabolische Sollbruchstelle" eingebaut wird, der aus einem oder mehreren C-Atomen in Kombination mit einem oder mehreren O- oder N-Atomen besteht (Fig. 2).

Im einfachsten Fall kann dieser Spacer aus mindestens einem Heteroatom, wie z. B. Sauerstoff oder Stickstoff, in Kombination mit einem C-Atom bestehen. Bevorzugte Kettenlängen des intramembranären Spacers sind 1-4 Atome.

Bei der Synthese eines derartigen "head-to-head"-Dimers ist nicht von den originären Fettsäure-Molekülen auszugehen, sondern von den ω-Hydroxy-Derivaten, z.B. der ω-Hydroxypalmitinsäure. Die Dimerisierung über das ω-ständige Kohlenstoffatom führt hier nicht zu einer reinen Kohlenwasserstoff-kette, wie in Fig. 2a gezeigt, sondern unter Wasseraustritt zu einer Sauerstoffverbrückung.

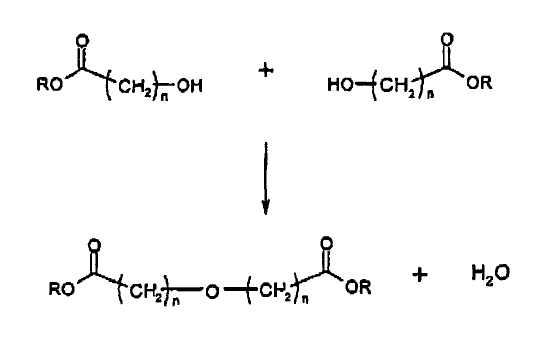

Das nun entstandene dimere Molekül enthält an der Verbrückungsstelle ein Sauerstoffatom. Die in Nachbarschaft stehenden Kohlenstoffatome (die originären ω-C-Atome) können enzymatisch hydroxyliert werden, etwa durch die Cytochrom-P₄₅₀-abhängigen mischfunktionellen Monooxygenasen. Eine derartige, in unmittelbarer Nachbarschaft zum O-Atom stattfindende Hydroxylierung führt zur Bildung instabiler Verbindungen mit Halbacetalstruktur, die in die entsprechenden Reaktionsprodukte zerfallen. Das eine Reaktionsprodukt mit ω-ständiger OH-Gruppe ist iden-tisch mit dem Ausgangsprodukt ω-Hydroxypalmitinsäure. Das andere Reaktionsprodukt ist eine Fettsäure mit ω-ständiger Aldehydfunktion, die zur Carbonsäuregruppe weiteroxidiert wird. Damit wird offensichtlich, dass durch einen (verlangsamt ablaufenden) biochemischen Abbau der beschriebenen dimeren und letztendlich auch der oligomeren Moleküle z. T. die physiologischen Ausgangsverbindungen selbst oder den Ausgangsverbindungen zumindest sehr ähnlich gebaute Moleküle entstehen.

Für den Fall, dass die Dimerisierung der beiden Fettsäure-Moleküle gleichzeitig zu einem kontrollierbaren Ausmaß der Abbaubarkeit des entstehenden dimeren Moleküls führen soll oder/und dass - etwa aus sterischen Gründen - das entstehende dimere Molekül eine längere Kette haben soll als der Summe der Kettenlängen der monomeren Moleküle entspricht, kann ein längerer intra-membranärer Spacer zwischen die beiden Fettsäurereste eingebaut werden. Dies wird z.B. durch die Verwendung von Glycolen, im einfachsten Fall von Ethylenglycol, zur Verbrückung von ω-Hydroxyfettsäuren erreicht. In diesem Fall entsteht ein Reaktionsprodukt, das zwei Sauerstoffatome in der Gesamtkette enthält:

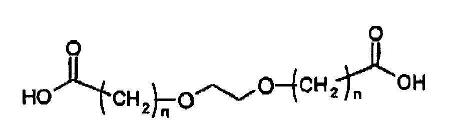

Das Gesamtmolekül ist damit gegenüber der Summe der beiden monomeren Moleküle praktisch um die Länge des intramembranären Spacers -O-CH₂-CH₂-O- gewachsen. Durch die beiden in dieser Kette vorhandenen Sauerstoffatome ist infolge der Oxidierbarkeit der den O-Atomen benachbarten C-Atome, ggf. noch durch zusätzlich Variation der Spacerstruktur, eine steuerbare Abbaugeschwindigkeit des dimeren und damit auch des oligomeren Moleküls gegeben.

Auf diese Weise kann durch die Wahl eines geeigneten intramembranären Spacers sowohl die Gesamtgröße des entstehenden dimeren Moleküls als auch das Ausmaß seiner biochemischen Abbaubarkeit frei gewählt werden, weil es möglich ist, sog. "metabolische Sollbruchstellen" in den intramembranären Spacer einzubauen. Allerdings ist darauf zu achten, dass ein intramembranärer Spacer keine ausgeprägten hydrophilen Eigenschaften besitzen sollte, da sonst die Möglichkeit der Integration des Dimers in die Lipid-Doppelmembran verringert werden kann.

Ein wesentlicher Aspekt der Pathogenese der oben genannten Hautveränderungen bzw. Hauterkrankungen ist die reduzierte Wasserbindefähigkeit des Hautgewebes, insbesondere im Bereich des Stratum corneum. Physiologischerweise wird das Wasser nicht innerhalb, sondern, da mehrere parallel angeordnete Lipid-Schichten vorliegen, in den Raum zwischen den einzelnen Lipid-Doppelschichten eingelagert. Dies liegt in der Tatsache begründet, dass das Innere der Lipid-Doppelschicht aus den stark hydrophoben Fettsäureresten aufgebaut ist, während das Medium außerhalb der Lipid-Doppelschicht hydrophiler Natur ist. Eine Speicherung von Wasser in den hydrophoben inneren Bereichen der Lipid-Doppelmembran ist praktisch nicht möglich.

Die anfangs genannten Hautveränderungen und -erkrankungen sind letztlich auf den Verlust eines Teiles der parallel angeordneten Lipid-Doppelschichten und der zwischen diesen Doppelschichten angeordneten hydrophilen Zwischenschichten sowie von Wasser zurückzuführen. Ziel der therapeutischen Maßnahmen bei diesen Erkrankungen ist somit nicht nur der Wiederaufbau und die Stabilisierung der Lipid-Doppelschichten selbst, wie dies mit Hilfe der oben beschriebenen Dimere verschiedener Fettsäuren erfolgt, sondern weiterhin auch der Aufbau und die Stabilisierung der multilamellaren Lipid-Strukturen mit den dazwischen liegenden hydrophilen Zwischenschichten, die für die Wasserbindefähigkeit der Haut von ausschlaggebender Bedeutung sind.

Dieses Ziel wird dadurch erreicht, dass mindestens zwei der oben erwähnten "tail-to-tail"-Fettsäure-Dimere kovalent verknüpft werden. Im Gegensatz zu der oben beschriebenen Bildung dimerer Fettsäure-Moleküle durch Schaffung einer kovalenten Bindung im hydrophoben Bereich des Moleküls, i. e. am ω-ständigen C-Atom der Fettsäurekette, erfolgt die kovalente Verbindung zweier Fettsäure-Dimere nach einem anderen Prinzip:
1. Die Verknüpfung zweier Fettsäure-Dimere erfolgt an den hydrophilen Enden der betreffenden Moleküle. Wie die Darstellung des Beispiels Palmitinsäure in Fig. 1 zeigt, steht am hydrophilen Ende des Moleküls jeweils eine COOH-Gruppe zur Verfügung, an welcher der Aufbau größerer Moleküle, bestehend aus mindestens zwei Fettsäure-Dimeren, erfolgen kann.
2. Die Verknüpfung zweier Fettsäure-Dimerer erfolgt nicht direkt, was unter Wasseraustritt durch Ausbildung einer (jedoch instabilen) Säureanhydrid-Gruppierung formal möglich wäre. Vielmehr ist es aus physiologischen Gründen notwendig, zwischen jeweils zwei sich ausbilden-den parallel angeordneten Lipid-Schichten einen Zwischenraum einer definierten Mindestgröße entstehen zu lassen, in welchen sich Wasser und ggf. hydrophile Moleküle, möglicherweise auch das vergleichsweise große Molekül Collagen, einlagern können. Der Aufbau eines Zwischen-raumes ist jedoch dann und nur dann möglich, wenn, wie oben bereits ausgeführt, die beiden zu verknüpfenden Fettsäure-Dimere durch einen extramembranären Spacer auf Abstand gehalten werden (Fig. 3).

Aus den oben erwähnten Gründen - der durch den Spacer geschaffene Zwischenraum zwischen zwei parallel angeordneten Lipid-Doppelschichten soll Wasser und hydrophile Moleküle aufnehmen und speichern können - muss der extramembranäre Spacer stets hydrophile Eigenschaften besitzen. Er kann jedoch je nach vorliegender oben erwähnter Hauterkrankung mit stark unterschiedlicher Kettenlänge ausgestattet sein.

Im folgenden sollen einige Beispiele von Strukturen extramembranärer Spacer angegeben werden, bei denen jeweils ein hydrophiler Molekülbaustein mit unterschiedlicher Struktur und Kettenlänge mit der jeweils am hydrophilen Ende des Fettsäure-Moleküls vorhandenen COOH-Gruppe verknüpft ist (in den folgenden Beispielen ist jeweils zusätzlich die nächstfolgende CH₂-Gruppe der beiden Fettsäure-Komponenten angegeben) :

Mit Glycerin als Spacer-bildendem Molekül ergibt sich die folgende Struktur des extramembranären Spacers mit zwei Estergruppierungen:

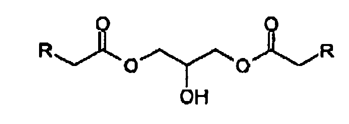

Der zusätzliche Einbau einer oder zweier beliebiger Aminosäuren, wie z. B. der Asparaginsäure, führt unter Ausbildung zweier Peptidbindungen und zweier Estergruppierungen zu einem verlängerten, wegen der Anwesenheit zweier freier, dissoziierter Carboxylgruppen stark hydrophilen Spacer:

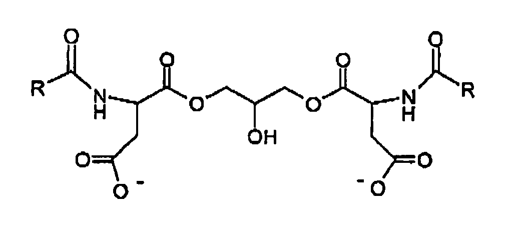

Bei der Verwendung von zwei Molekülen Serin ergibt sich unter Ausbildung von vier Estergruppierungen ein verlängerter Spacer, der wegen der Anwesenheit zweier
bei physiologischen pH-Werten protonierbarer Aminogruppen auch stark hydrophil ist: 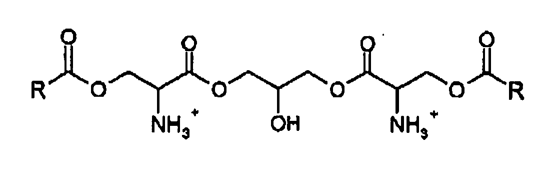 Von besonderem Interesse ist der Aufbau eines Harnstoff-Derivats als Strukturelement des extramembranären Spacers. Dies ist z.B. durch den Einsatz zweier Moleküle einer basischen Aminosäure wie z.B. Lysin möglich. Hierbei entsteht ein relativ langer Spacer mit stark hydrophiler Natur, die u. a. durch die Anwesenheit der beiden negativ geladenen Carboxylgruppen gegeben ist:

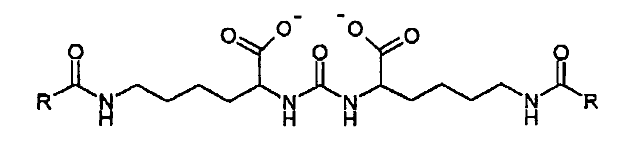

Der Aufbau einer Harnstoff-ähnlichen Struktur ist deswegen von besonderem Interesse, weil Harnstoff über ein sehr hohes Wasserbindevermögen verfügt, was heute bereits in Form Harnstoff-haltiger Salben zur Therapie von solchen Hauterkrankungen genutzt wird, bei denen ein Austrocknen der Haut ein wesentliches Krankheitsmerkmal darstellt (z.B. beim dyshidrotischen Ekzem).

Der Vielfalt der Spacer-Strukturen und der Länge der verwendbaren Spacer sind praktisch keine Grenzen gesetzt. Die Struktur wie auch die Kettenlänge können je nach Bedarf der speziellen Therapieanforderungen in weiten Bereichen variiert werden. Möglich ist u. a. auch der Einbau bestimmter Monosaccharide wie z.B. von Glucose, was wiederum zu Derivaten physiologischer Substanzen führt.

Die Wahl der genannten verschiedenen Strukturen im interdimeren Spacer führt zu unterschiedlichen biologischen Stabilitäten und damit zu einem unterschiedlichen Ausmaß der gewünschten Abbaubarkeit des Fettsäure-Moleküls, die einerseits deutlich unter dem Wert für die monomeren Fettsäure-Moleküle liegen soll, andererseits jedoch nicht völlig fehlen soll. Damit ist auch über diese intermolekulare Spacerstruktur eine gewisse Steuerbarkeit der Wirkungsstärke und -dauer der zur Therapie eingesetzten Fettsäure-Dimere gegeben.

Auch beim metabolischen Abbau der beschriebenen Fettsäure-Moleküle, insbesondere ihrer Spacer, entstehen Abbauprodukte, die identisch mit physiologischen Substanzen sind (z.B. Aminosäuren + monomere Fettsäuren) oder eine sehr große Ähnlichkeit mit ihnen besitzen, so dass die Wahrscheinlichkeit unerwünschter Nebenwirkungen, insb. toxischer Wirkungen, denkbar gering ist.

Gegenüber den dimeren Fettsäure-Molekülen weisen Dimere-enthaltend oligomere Moleküle mit 2-12 Monomeren, insb. Tetramere, Hexamere oder Octamere, eine größere Fähigkeit auf, die Struktur der parallel angeordneten Lipid-Membran-Doppelschichten zu stabilisieren. Durch diese Verbindungen kommt es zum Aufbau bzw. zur Stabilisierung von 2 parallel angeordneten Doppelschichten im Fall der Tetrameren, von 3 parallel angeordneten Doppelschichten im Fall der Hexameren, von 4 parallel angeordneten Doppelschichten im Fall der Octameren, etc., mit einer verstärkten Tendenz zur Einlagerung von Wasser und hydrophilen Molekülen unterschiedlichster Größe in die Räume zwischen die parallelen Lipid-Schichten.

Oligomere Fettsäuren mit einer ungeraden Zahl von Molekülen, im einfachsten Fall also ein trimeres Molekül mit einem intramembranären und einem extramembranären Spacer, sind für die hier angesprochenen Zwecken durchaus auch einsetzbar, wenn sie auch nicht über die optimalen Eigenschaften zur Integration in die vorhandenen Lipid-Doppelschichten verfügen. Im Beispiel eines trimeren Fettsäure-Moleküls würden sich die beiden über einen intramembranären Spacer verbundenen Fettsäure-Moleküle in eine Lipid-Doppelschicht optimal integrieren, während das über einen extramembranären spacer verbundene weitere Fettsäure-Molekül lediglich in eine Hälfte der nächsten Lipid-Doppelschicht hineinragt.

Einen Sonderfall stellt ein Fettsäure-Dimeres dar, das über einen extramembranären Spacer verbunden ist (gemäß der oben angegebenen "head-to-head"-Variante der Verknüpfung bei der Fettsäure-Dimerisierung). Auch für diese Moleküle gilt, dass sie für die hier angesprochenen Zwecke durchaus einsetzbar sind, wenn sie auch noch weniger über die optimalen Eigenschaften zur Integration in die vorhandenen Lipid-Doppelschichten verfügen. In diesem Fall ragen beide vorhandene Fettsäure-Moleküle lediglich in eine Hälfte der jeweils benachbarten Lipid-Doppelschichten hinein.

Die Erfindung wird nachfolgend anhand der Figuren und Beispiele näher erläutert. Diese sollen jedoch die vorliegende Erfindung nicht auf die hier gezeigten Ausführungsformen beschränken.
- Fig. 1: zeigt die Anordnung einer freien Fettsäure in einer typischen stabilen Struktur der Lipid-Doppelschicht biologischer Membranen. Die hydrophoben Fettsäurereste sind in das Innere der Membran orientiert und bilden dort einen hydrophoben Bereich. Die hydrophilen, polaren Reste sind nach außen in Richtung der wässrigen Phase, d.h. der angrenzenden Intra- bzw. Extrazellular- flüssigkeit, ausgerichtet.
- Fig. 2: zeigt die erfindungsgemäße Kopplung zweier geradkettiger unverzweigter Fettsäuren zu einem Dimeren durch Verknüpfung mit einem intramembranären Spacer (Rechteck).
- Fig. 3: zeigt in schematischer Darstellung die An- ordnung eines nicht erfindungsgemäßen "head-to-head"-Dimeren zweier geradkettiger unver-zweigter Fettsäuren durch Verknüpfung mit einem extramembranären Spacer. Das Di- mer ist mit je einem Monomer in jeweils ei- ner Hälfte zweier parallel angeordneter Li- pid-Doppelschichten verankert. Das Rechteck zwischen den beiden Monomeren stellt den sog. extramembranären Spacer dar. Darge- stellt ist jeweils nur eine Hälfte der bei- den benachbarten Doppelmembranen.
- Fig. 4: zeigt in schematischer Darstellung die An- ordnung nicht erfindungsgemäßer tetramerer Moleküle dar, die als verbindendes Element zwischen zwei Lipid-Doppelschichten fungie- ren. Zwischen den beiden benachbarten Li- pid-Doppelschichten liegt ein hydrophiler Zwischenraum. Die beiden in der "tail-to- tail"-Anordnung aufgebauten Dimere, die im Innern der Membran liegen, sind durch einen intramembranären Spacer verbunden. Die bei- den Dimere selbst sind über einen extra- membranären Spacer verknüpft, der sich im hydrophilen Zwischenraum der beiden Membra- nen befindet.

In Analogie zu den in Fig. 4 gezeigten tetrameren Verbindungen können sich in gleicher Weise Hexamere in drei voneinander getrennte Lipidmembranen, Octamere in vier Lipidmembranen, usw., einlagern. Fettsäure-Oligomere der beschriebenen Art können in der Medizin zu therapeutischen Zwecken überfall dort angewandt werden, wo der natürliche Aufbau biologischer Membranen durch pathologische Vorgänge gestört ist und sich durch den Einsatz dieser oligomeren Verbindungen eine Stabilisierung der Membranstruktur und/oder eine Veränderung der Membraneigenschaften im Sinne eines therapeutischen ziels (z. B. zur Erhöhung der Membranstabilität) erreicht werden soll.

Nachfolgend einige Beispiele:
- Im Fall bestimmter Vergiftungen, welche vorzugsweise die Leber betreffen, wie z.B. eine Vergiftung mit Tetrachlormethan (Tetrachlorkohlenstoff, TETRA, CCl₄), werden die Lipide der Leberzellmembranen durch Radikale in ihrer Struktur angegriffen. Bei diesem Vorgang werden die Fettsäurereste der Lipide oxidiert, wodurch nach einer Reihe verschiedener Reaktionen die Kohlenstoffkette abgebaut wird. Die Folge hiervon ist ein partieller Abbau der Lipide und eine Destabilisierung der Membran, die zu einer teilweisen Auflösung der Zellmembran und damit einer schweren Schädigung der Zelle führt. Die Zufuhr der beschriebenen erfindungsgemäßen Verbindungen, im vorliegenden Fall von Fettsäure-Dimeren, kann in einem derartigen Vergiftungsfall zu einer deutlichen Stabilisierung der Membran der geschädigten Leberzellen beitragen.
- Eine Veränderung der Lipid-Zusammensetzung von Nervenzellen tritt bei einer großen Zahl unterschiedlicher pathologischer Schädigungen von Nervenzellen auf. Hierzu gehören u. a. die Neuronopathie, die Axonopathie und die Myelinopathie. Als Ursachen für die Schädigung bzw. den Abbau der Lipid-reichen Myelinscheiden gilt u.a. die Einwirkung exogener Schadstoffe.
- Im Fall von Myelinopathien wie z.B. der Multiplen Sklerose kommen für eine Stabilisierung der LipidMembranen der Myelinscheiden wegen deren spezifischer Struktur vorzugsweise Dimere-enthaltende Oligomere der genannten Fettsäuren sowohl mit kurzen hydrophoben intramembranären als auch mit kurzen hydrophilen extramembranären Spacern in Betracht.
- In mehreren Untersuchungen konnte bisher nachgewiesen werden, dass die Anwesenheit von ω-3-mehrfach-ungesättigten Fettsäuren in Lipiden eine antithrombotische Wirkung besitzt. Hintergrund dieser Wirkung ist offensichtlich die bevorzugte Einlagerung dieser Lipid-Spezies in die Membranen von Blutzellen, insbesondere in Membranen der Blutplättchen, gegenüber derjenigen der Lipide mit ω-6-mehrfach-ungesättigten Fettsäuren. Die Anwendung von Dimere-enthaltenden Fettsäure-Oligomeren mit einem hohen Gehalt von ω-3-mehrfach-ungesättigten Fettsäuren bietet sich insb. in denjenigen Fällen an, in denen eine genetisch determinierte Fettstoffwechselstörung zu einem hohen thrombotischen, atherosklerotischen und cardiovaskulären Risiko führt.
- Nach derzeitiger Kenntnis sind Hauterkrankungen eines der Hauptgebiete für den Einsatz der genannten Dimere-enthaltenden Fettsäure-Oligomere, nicht zuletzt deswegen, weil im Stratum corneum der menschlichen Haut geradkettige, unverzweigte Fettsäuren, insb. Palmitinsäure, eine wesentliche Rolle spielen.

Die genannten Verbindungen können beispielsweise über die folgenden Wege synthetisiert werden:

### 1. Dimerisierung mit intramembranärem Spacer

Der intramembranäre Spacer muss wegen seiner Lage im Innern der biologischen Membran überwiegend hydrophobe Eigenschaften besitzen. Er hat die folgende allgemeine Struktur: 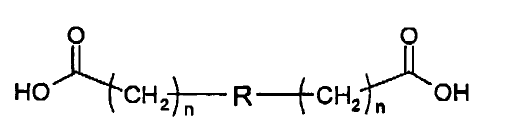
R = O, N, -O- (CH₂)ₓ-O- x = 1 - 30

Bei Oligomeren mit mehr als 2 Fettsäuren kann auch
R = - (CH₂)_{y}- sein mit y = 1 -30

### Beispiele für Synthesewege:

### 1.1 Kondensation zweier ω-Hydroxycarbonsäureester mit nachfolgender Esterspaltung:

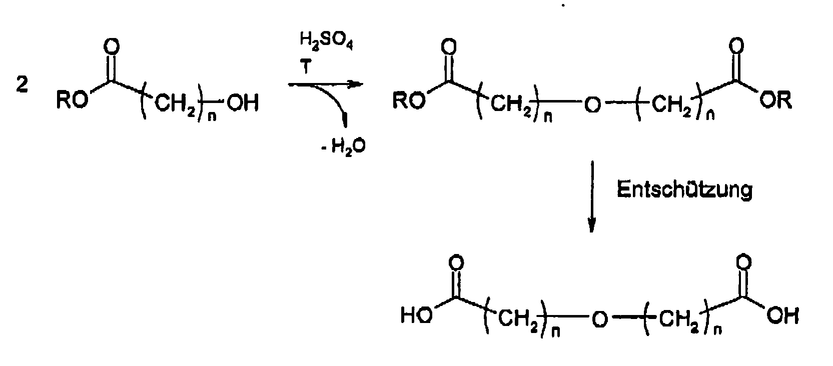

### 1.2 Ethersynthese mit ω-Hydroxycarbonsäureestern und ω-Halogencarbonsäureestern mit nachfolgender Esterspaltung:

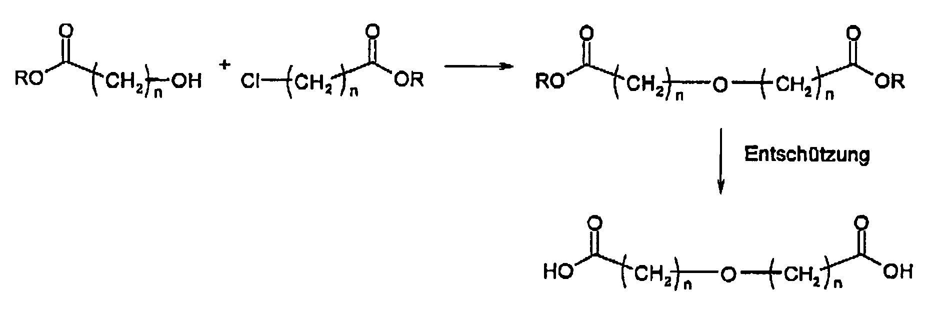

Bei Oligomeren mit mehr als 2 Fettsäuren kann auch R = -(CH₂)_{y}- sein. Dann ergeben sich die folgenden Synthesemöglichkeiten:
1.3 C-C-Knüpfung zweier ω-Halogencarbonsäureester mit nachfolgender Esterspaltung 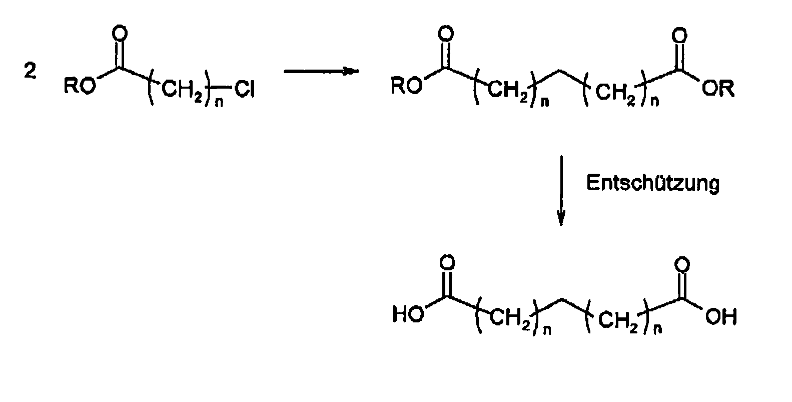
1.4 C-C-Knüpfung zweier ω-Aldehyd-carbonsäuren mit nachfolgender Entschützung 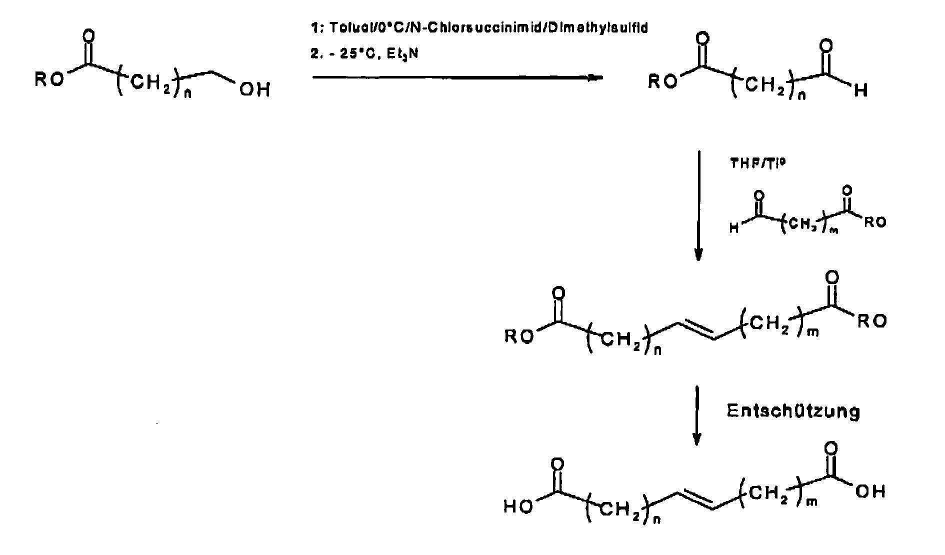
1.5 C-C-Verknüpfung mit jeweils nachfolgender Reaktion zur Dicarbonsäure
1.5.1 C-C-Verknüpfung zweier ω-Halogen-1-alkene: 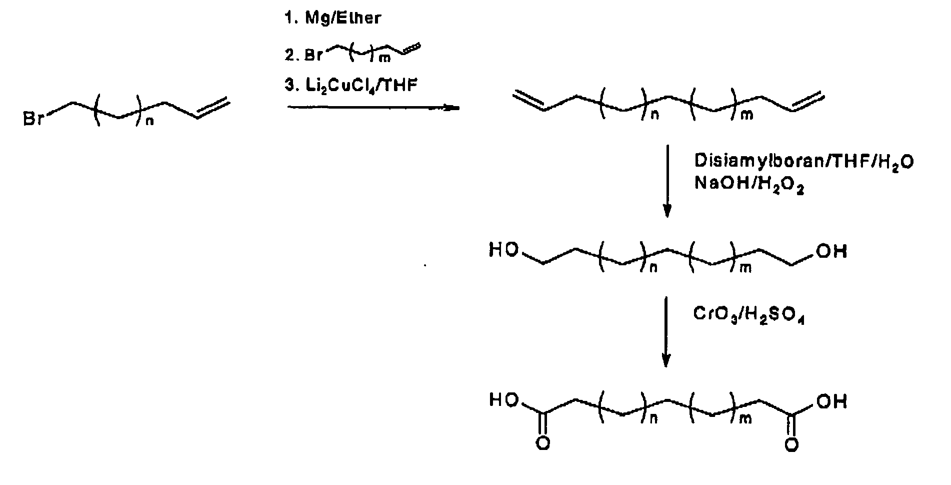
1.5.2 C-C-Verknüpfung zweier Carbon-ω-en-säuren mit 1, ω-Dibromalkan 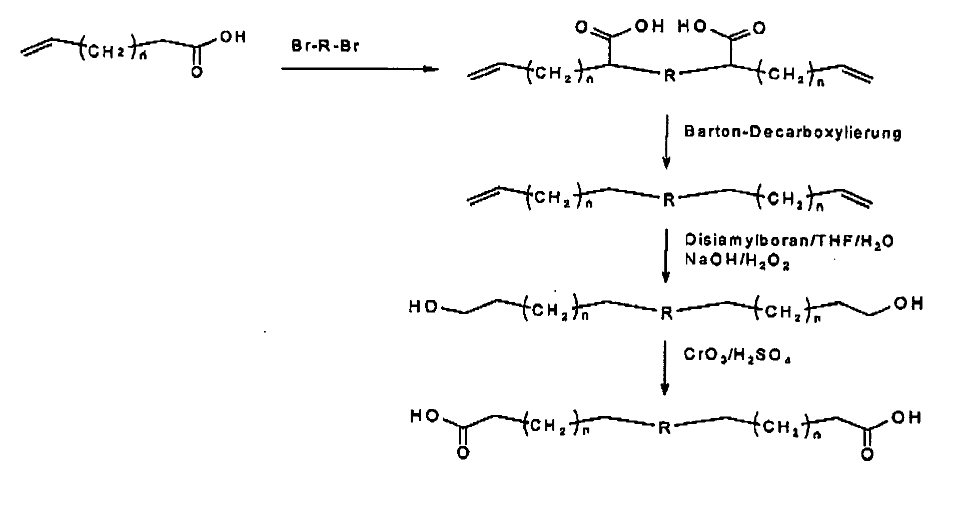
1.5.3 Darstellung über ω-Hydroxy-1-alkene bzw. ω-Halogen-1-alkene mit 3,4-O-Isopropyliden-D-Mannitol 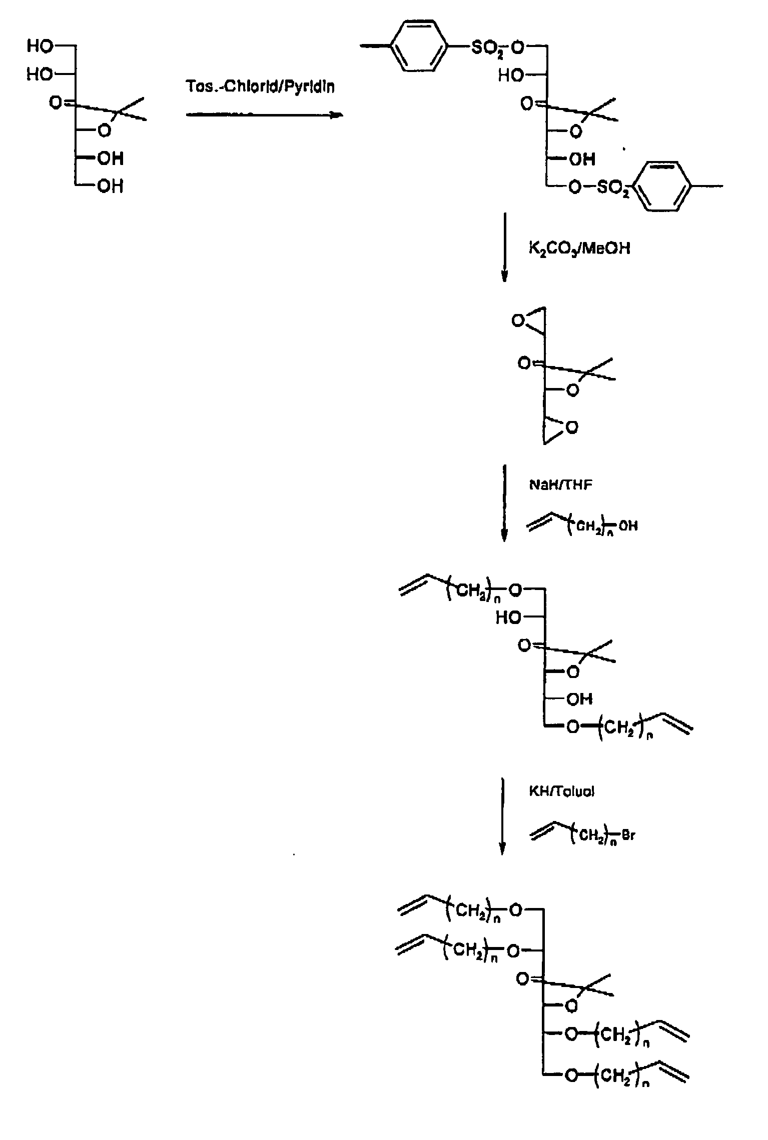 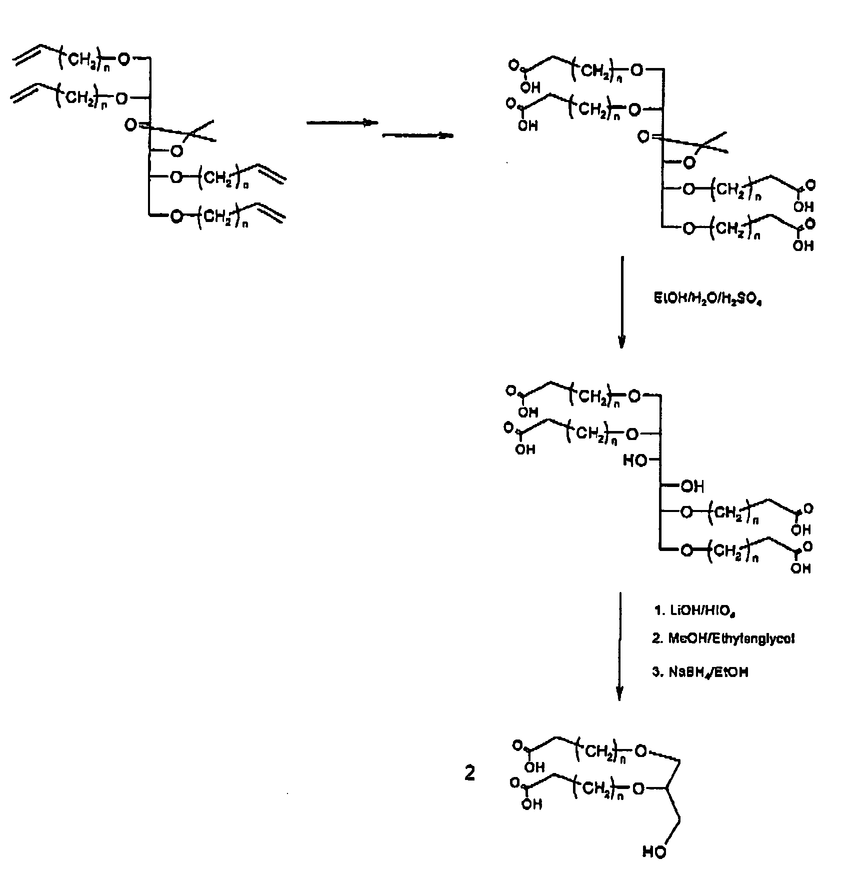
1.6 Diethersynthese mit ω-Hydxoxycarbonsäureestern und einem 1,ω-Dibromalkan mit folgender Esterspaltung:

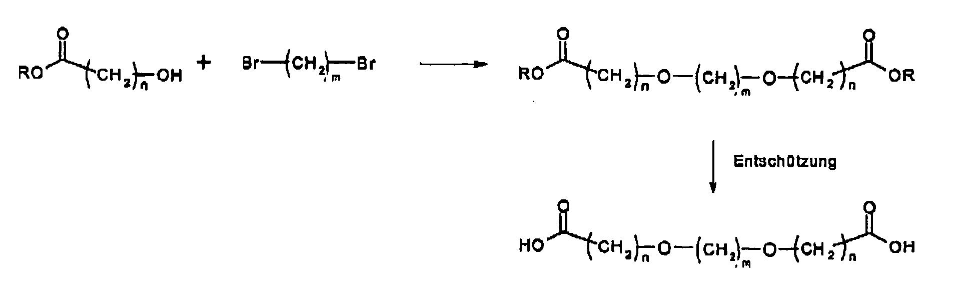

### 2. Dimerisierung/Oligomerisierung mit extramembranärem Spacer

Der extramembranäre Spacer muss wegen seiner Lage außerhalb biologischer Membranen überwiegend hydrophile Eigenschaften besitzen. Er hat die folgende allgemeine Struktur:

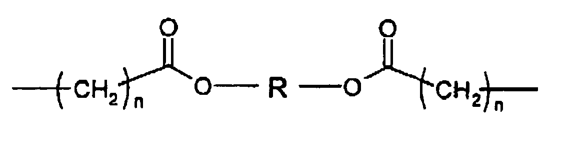

Beispiele für Synthesewege:
2.1 Synthese von Estern aus zwei Fettsäure (n, chloriden, amiden, estern) mit bifunktionellen Verbindungen (Spacer-Ausgangsmolekülen): 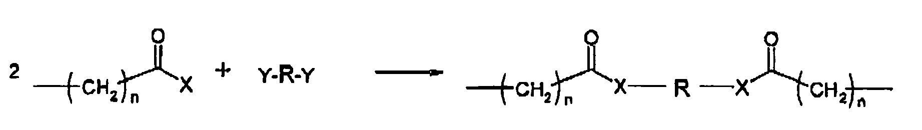
2.2 Schrittweise Verknüpfung zu Fettsäurediestern
2.2.0 Allgemeines Reaktionsschema:

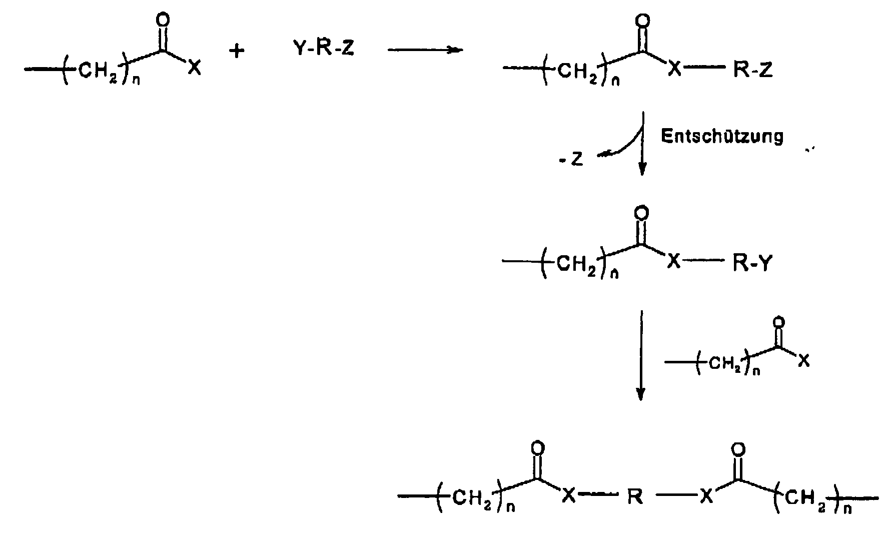

**Tabelle 1: Verknüpfungs-Moleküle (Spacer-Ausgangaverbindungen) Y-R-Y mit Y = OH, Cl, Br, NH₂, NHR, OR' (Alkan, Alken, Alkin, Aren) oder mit -O-, -N- und mit Z = als Schutzgruppe (vgl. Tabelle 2)**

| **R** | **Y** | **Y-R-Y** |
|---|---|---|
| 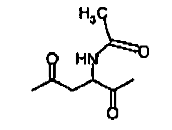 | OH | N-Acetyl-asparaginsäure |
| 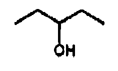 | Cl, NH₂ | 1,3-Dichlor-2-propanol 1,3-Diamino-2-propanol |
| 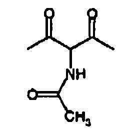 | OEt | Diethylacetoamidomalonat |
| 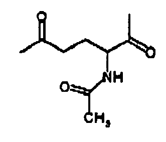 | OH | N-Acetyl-glutaminsäure |
| 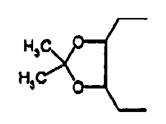 | 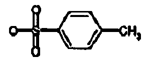 | 2,3-O-Isopropylideden 1,4 di-O-tosyl D-threit |
| 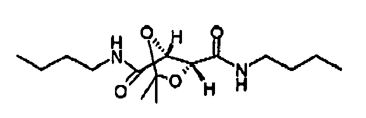 | Br, OH | N,N'-Dihydroxypropanyl-2,3-isopropyliden-tatrat, N,N'-Dibrompropanyl-2,3-isopropyliden-tatrat; (aus N,N'-Diallyl-weinsäure-diamid |
| 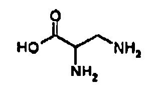 | NH₂ | D-2,3-Diamino-propionsäure |
| 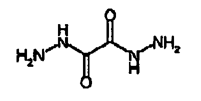 | NH₂ | Oxalsäuredihydrazin |
| 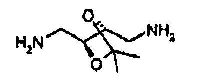 | NH₂ | Diamino-2,3-isopropyliden-butan |
| 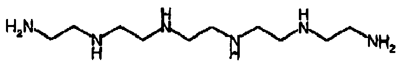 | NH₂ | Pentaethylenhexamin |
| 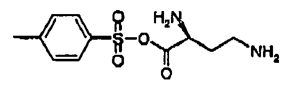 | NH₂ | 2,4-Diamino-O-tosylbuttersäureester |
| 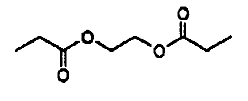 | Cl | Ethylenglycol-bis-chloracetat |
| 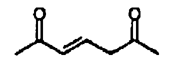 | OMe | Dimethylglutaconat |
| 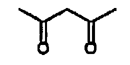 | OEt, Cl | Ethyl-4-chloracetoacetat |
| 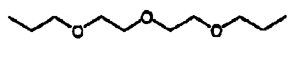 | OH, Cl | Tetraethylenglycol |
| 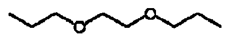 | OH, Cl, NH₂ | Triethylenglycol, 1,2-Bis-(2-chlorethoxy)ethan 1,2-Bis-(2-aminoethoxy)ethan |
| 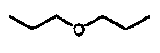 | Cl | Bis (2-chlorethyl)-ether |
| 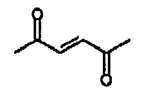 | Cl | Fumarsäuredichlorid |
| 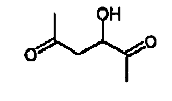 | OH, OMe | Apfelsäure, Dimethylmalat |
| 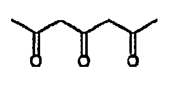 | OEt, OH | Diethyl-3-oxoglutarat, 3-Oxoglutarsäure |
| 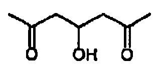 | OEt | Diethyl-3-hydroxyglutarat |
| 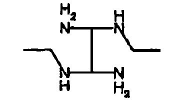 | | Acetylendiharnstoff |
| 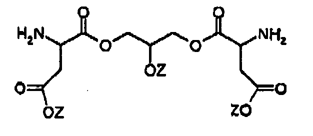 | NH₂ | Bis- (tosyl-asparginsäureester)-2-benzylglycol |
| 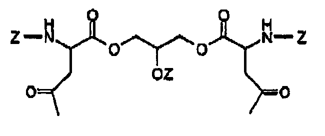 | OH, | Bis-(N-Boc-asparginsäure) -2-benzylglycol |
| 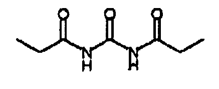 | Cl | N,N'-(3-chlorpropyryl)-harnstoff |
| 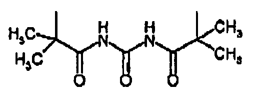 | Br | N,N'-(2-Bromisobutyryl) harnstoff |
| 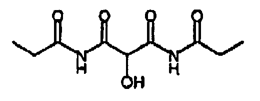 | Cl | N-(3-chlorpropyryl)-2-hydroxy-glutaratamid |
| 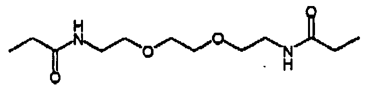 | Cl | 1,2-Bis-[2-N-(3-chlorpropyryl)-aminoethoxy)-ethan |
| 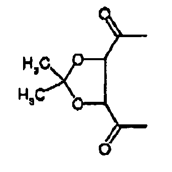 | OMe | Dimethyl-2,3-isopropylidentatrat |
| 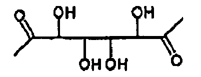 | OH | Schleimsäure |
| 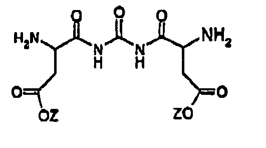 | NH₂ | N,N'-(Asparginsäure-benzylester)-harnstoff |
| 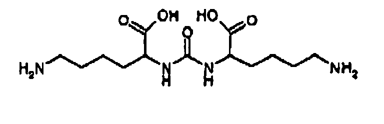 | NH₂, Br, Cl, OH | N,N'-(1-Carboxy-5-aminopropan)-harnstoff, N,N'-(1-Carboxy-5-brom-propan)-harnstoff, N,N'-(1-Carboxy-5-Chlorpropan)-harnstoff, N,N'-(1-Carboxy-5-hydroxy-propan)-harnstoff |

2.2.1 Reaktion eines Säurechlorids mit 2,3-Isopropyliden-snglycerol

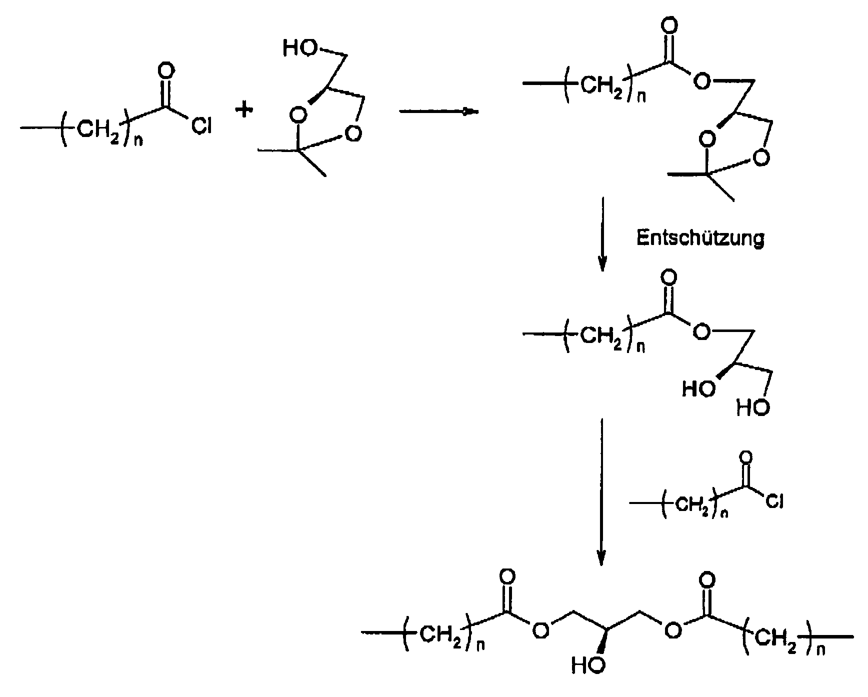

Hieran schließen sich die folgenden Folgereaktionen an:
2.2.1.1 Umwandlung von Alkoholen zu Aminen: 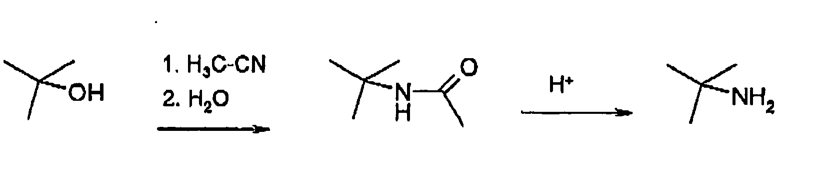
2.2.1.2 Umwandlung von Halogenverbindungen zu Carbonsäuren:

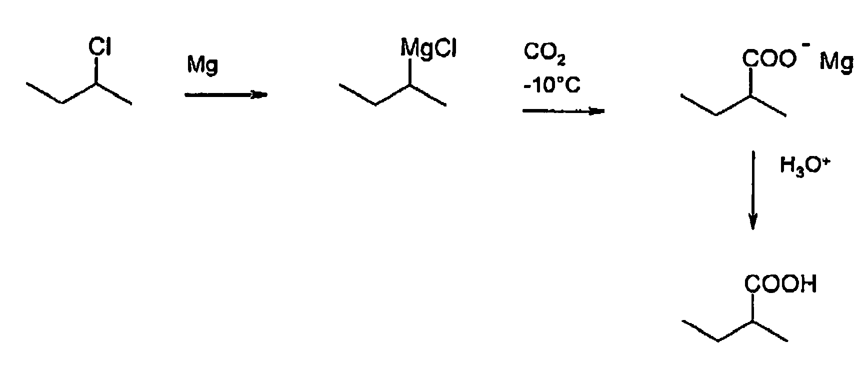

### 2.2.1.3 Umwandlung von Säurechloriden zu Harnstoffderivaten

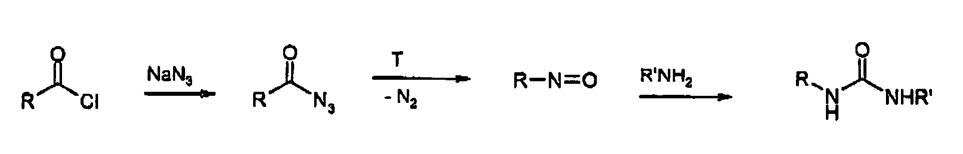

### 2.2.1.4 Umwandlung von α-Ketonsäuren zu α-Aminocarbonsäuren

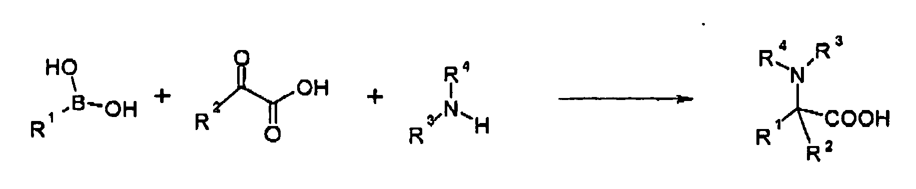

### 2.2.2 Schutzgruppen

Bei den beschriebenen synthesareaktionen können als Schutzgruppen (Z) die in Tabelle 2 genannten Verbindungen eingesetzt werden

**Tabelle 2: Einsetzbare Schutzgruppen(Z):**

| | |
|---|---|
| Für Alkohole: | |
| 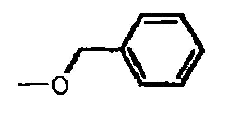 | Benzyl-Gruppe |
| 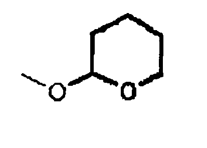 | THP-Gruppe |
| 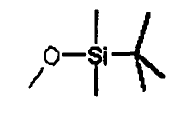 | tert-Butyldimethylsilyl-Gruppe |
| 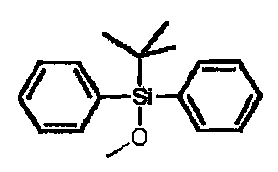 | tert-Butyldiphenylsilyl-Gruppe |
| Für Amine: | |
| 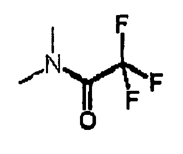 | Trifluoracetat-Gruppe |

| | |
|---|---|
| 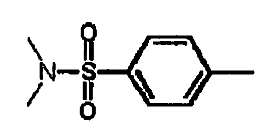 | Tosyl-Gruppe |
| 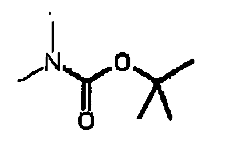 | Boc-Gruppe |
| 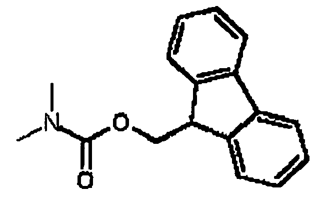 | Fmoc-Gruppe |
| 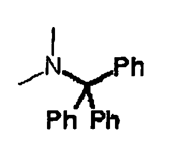 | Trityl-Gruppe |
| Für Carbonsäuren: | |
| 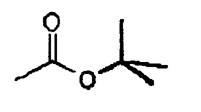 | tert-Butyl-Gruppe |
| 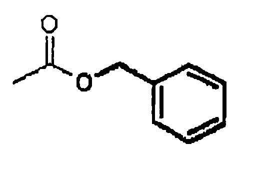 | Benzyl-Gruppe |

## Patentansprüche

1. Kosmetik- oder Körperpflegemittel enthaltend ein Dimer von mindestens zwei geradkettigen und unverzweigten Fettsäuren, wobei die Fettsäuren jeweils über deren hydrophobes Ende durch Verknüpfung der beiden ω-ständigen C-Atome über einen heterätomhaltigen Spacer kovalent aneinander gebunden sind, wobei der Spacer hydrophob ist.

2. Kosmetik- oder Körperpflegemittel nach Anspruch 1,
**dadurch gekennzeichnet, dass** die Fettsäuren unabhängig voneinander aus Monocarbonsäuren mit einer Kettenlänge zwischen 10 und 40 C-Atomen ausgewählt sind.

3. Kosmetik- oder Körperpflegemittel nach Anspruch 2,
**dadurch gekennzeichnet, dass** die Fettsäuren ausgewählt sind aus der Gruppe bestehend aus Palmitinsäure, Laurinsäure, Myristinsäure, Stearinsäure, Arachinsäure, Lignocerinsäure, Palmitoleinsäure, Ölsäure, 9,11-Octadecadiensäure (C₁₇H₃₁-COOH), Linolsäure, Linolensäure, 5,8,11,14,17-Eicosapentaensäure, 4,7,10,13,16,19-Docosahexaensäure, Cerebronsäure, Decansäure, Octacosansäure und 9-Octacosensäure.

4. Kosmetik- oder Körperpflegemittel nach einem der vorhergehenden Ansprüche,
**dadurch gekennzeichnet, dass** der Spacer intramembranär ist und aus einem oder mehreren C-Atomen in Kombination mit einem oder mehreren O- oder N-Atomen besteht.

5. Kosmetik- oder Körperpflegemittel nach einem der vorhergehenden Ansprüche,
**dadurch gekennzeichnet, dass** mindestens eine Fettsäure gesättigt ist.

6. Kosmetik- oder Körperpflegemittel nach einem der vorhergehenden Ansprüche,
**dadurch gekennzeichnet, dass** mindestens eine Fettsäure ungesättigt ist,

7. Kosmetik- oder Körperpflegemittel nach einem der vorhergehenden Ansprüche,
**dadurch gekennzeichnet, dass** die Fettsäuren natürlichen, halbsynthetischen oder synthetischen Ursprungs sind.

8. Kosmetik- oder Körperpflegemittel nach einem der Ansprüche 1 bis 7 als Human- oder Veterinärarzneimittel.

9. Verwendung des Kosmetik- oder Körperpflegemittels nach einem der Ansprüche 1 bis 7 zur Herstellung eines Arzneimittels für die Prophylaxe und Therapie von Erkrankungen, bei denen eine Störung der Lipidzusammensetzung der Zellmembranen der Haut hinsichtlich ihres Gehalts an Fettsäuren vorliegt.

10. Verwendung nach Anspruch 9,
**dadurch gekennzeichnet, dass** eine Störung der Zusammensetzung der Lipid-Doppelschichten des Stratum corneum der Haut hinsichtlich ihres Gehalts an Fettsäuren vorliegt.

## Claims

1. A cosmetic or body care product containing a dimer of at least two straight-chain and unbranched fatty acids, the fatty acids being covalently bonded together in each case via their hydrophobic ends by linking the two C atoms in the ω position via a heteroatom-containing spacer, the spacer being hydrophobic.

2. A cosmetic or body care product according to Claim 1, **characterised in that** the fatty acids independently of each other are selected from monocarboxylic acids with a chain length of between 10 and 40 C atoms.

3. A cosmetic or body care product according to Claim 2, **characterised in that** the fatty acids are selected from the group consisting of palmitic acid, lauric acid, myristic acid, stearic acid, eicosanoic acid, tetracosanoic acid, palmitoleic acid, oleic acid, octadeca-9,11-dienoic acid (C₁₇H₃₁-COOH), linoleic acid, linolenic acid, eicosa-5,8,11,14,17-pentaenoic acid, docosa-4,7,10,13,16,19-hexaenoic acid, cerebronic acid, decanoic acid, octacosanoic acid and octacos-9-enoic acid.

4. A cosmetic or body care product according to one of the preceding claims, **characterised in that** the spacer is an intramembrane spacer and consists of one or more C atoms in combination with one or more 0 or N atoms.

5. A cosmetic or body care product according to one of the preceding claims, **characterised in that** at least one fatty acid is saturated.

6. A cosmetic or body care product according to one of the preceding claims, **characterised in that** at least one fatty acid is unsaturated.

7. A cosmetic or body care product according to one of the preceding claims, **characterised in that** the fatty acids are of natural, semi-synthetic or synthetic origin.

8. A cosmetic or body care product according to one of Claims 1 to 7 as a human or veterinary medicament.

9. Use of the cosmetic or body care product according to one of Claims 1 to 7 for the production of a medicament for the prophylaxis and therapy of diseases in which a disorder of the lipid composition of the cell membranes of the skin in terms of its content of fatty acids is present.

10. Use according to Claim 9, **characterised in that** a disorder of the composition of the lipid double layers of the stratum corneum of the skin in terms of its content of fatty acids is present.

## Revendications

1. Produit cosmétique ou de soins corporels contenant un dimère d'au moins deux acides gras à chaîne linéaire et non ramifiée, les acides gras étant liés de façon covalente l'un à l'autre par leurs extrémités hydrophobes par un espaceur contenant un hétéroatome liant les deux atomes de carbone ω, l'espaceur étant hydrophobe.

2. Produit cosmétique ou de soins corporels selon la revendication 1, **caractérisé en ce que** les acides gras sont choisis indépendamment les uns des autres parmi des acides monocarboxyliques avec une longueur de chaîne comprise entre 10 et 40 atomes de carbone.

3. Produit cosmétique ou de soins corporels selon la revendication 2, **caractérisé en ce que** les acides gras sont choisis parmi le groupe consistant en l'acide palmitique, l'acide laurique, l'acide myristique, l'acide stéarique, l'acide arachidique, l'acide lignocérique, l'acide palmitoléique, l'acide oléique, l'acide 9,11-octadiénoïque (C₁₇H₃₁-COOH), l'acide linoléique, l'acide 5,8,11,14,17-eicosapentanoïque, l'acide 4,7,10,13,16,19-docosahexaénoïque, l'acide cérébronique, l'acide décanoïque, l'acide octacosanoïque et l'acide 9-octacosénoïque.

4. Produit cosmétique ou de soins corporels selon l'une quelconque des revendications précédentes, **caractérisé en ce que** l'espaceur est intramembranaire et consiste en un ou plusieurs atomes de carbone en combinaison avec un ou plusieurs atomes de O ou de N.

5. Produit cosmétique ou de soins corporels selon l'une quelconque des revendications précédentes, **caractérisé en ce qu'**au moins un acide gras est saturé.

6. Produit cosmétique ou de soins corporels selon l'une quelconque des revendications précédentes, **caractérisé en ce qu'**au moins un acide gras est insaturé.

7. Produit cosmétique ou de soins corporels selon l'une quelconque des revendications précédentes, **caractérisé en ce que** les acides gras sont d'origine naturelle, semi-synthétique ou synthétique.

8. Produit cosmétique ou de soins corporels selon l'une quelconque des revendications 1 à 7 comme médicament de médecine humaine ou vétérinaire.

9. Utilisation du produit cosmétique ou de soins corporels selon l'une quelconque des revendications 1 à 7 à la préparation d'un médicament pour la prophylaxie et la thérapie d'affections pour lesquelles il y a une perturbation de la composition en lipides des membranes cellulaires de la peau du point de vue de leur teneur en acides gras.

10. Utilisation selon la revendication 9, **caractérisée en ce qu'**il y a une perturbation de la composition des doubles couches lipidiques de la couche cornée de la peau du point de vue de leur teneur en acides gras.
